# EUROPÄISCHE PATENTSCHRIFT

(11) **EP 2 564 799 B1**
(45) Veröffentlichungstag und Bekanntmachung des Hinweises auf die Patenterteilung: **23.04.2014**
(21) Anmeldenummer: 12182250.6
(22) Anmeldetag: 29.08.2012
(51) Int. Cl.: A61B 17/86, A61B 17/72

(54) **Chirurgischer Mittelfuß-Kompressionsnagel**
Surgical metatarsal compression nail
Clou chirurgical de compression du métatarse

(30) Priorität: 31.08.2011 DE 102011053141
(43) Veröffentlichungstag der Anmeldung: 06.03.2013
(73) Patentinhaber: Zimmer GmbH, 8404 Winterthur (CH)
(72) Erfinder: Pech, Uwe, 78532 Tuttlingen (DE)
(74) Vertreter: Manitz, Finsterwald & Partner GbR

(56) Entgegenhaltungen:
- DE-T2- 60 204 657
- US-A- 5 964 768
- US-A- 6 030 162

## Beschreibung

Die Erfindung betrifft einen chirurgischen Mittelfuß-Kompressionsnagel zur Implantation im ersten Strang eines menschlichen Fußes gemäß dem Oberbegriff des Anspruchs 1, mit einem einen zum Eindrehen und/oder Ausdrehen dienenden Antrieb aufweisenden Kernbereich, einem vorderen Gewindeabschnitt mit einem vorderen Außengewinde, einem hinteren Gewindeabschnitt mit einem hinteren Außengewinde und mit einem zwischen den Gewindeabschnitten angeordneten Mittelabschnitt, wobei die Gewindesteigung des vorderen Außengewindes zur Erzielung einer Kompression größer ist als diejenige des hinteren Außengewindes.

Auf dem Markt bekannt ist u.a. ein Mittelfuß-Kompressionsnagel der Firma Synthes GmbH, der zur Behandlung von Mittel-/Hinterfußkollapsen eingesetzt wird. Der bekannte Kompressionsnagel weist eine gestuft zylindrische Hüllkontur auf und ist mit einem vorderen sowie einem hinteren Außengewinde versehen, wobei die Gewindesteigung des vorderen Außengewindes zum Erzielen einer Kompression größer ist als diejenige des hinteren Außengewindes. Der bekannte Kompressionsnagel umfasst einen gestuft ausgebildeten Kernbereich, mit einem langgestreckten hinteren Zylinderabschnitt und einem axial daran angrenzenden vorderen, durchmesserreduzierten Zylinderabschnitt, wobei am hinteren Zylinderabschnitt das hintere zylindrisch konturierte Außengewinde vorgesehen ist und gleichzeitig an das hintere Außengewinde angrenzend ein gewindefreier Mittelabschnitt vorgesehen ist. Der bekannte Mittelfuß-Kompressionsnagel ist hinsichtlich seiner Kompressionswirkung verbesserungsbedürftig.

Aus der FR 2 922 749 ist ein Kompressionsnagel mit zwei Gewindeabschnitten bekannt, wobei ein hinterer Gewindeabschnitt eine sich nach proximal verjüngende Hüllkontur aufweist. In einer zwischen den beiden Gewindeabschnitten befindet sich ein Mittelabschnitt, der durchgehend zylindrisch konturiert ist. Die Kompressionswirkung ist verbesserungswürdig.

Aus der DE 602 04 657 T2 ist eine Knochenschraube bekannt, die zwei axial beabstandete Gewindeabschnitte aufweist und einen zwischen den Gewindeabschnitten vorgesehenen Mittelabschnitt, der axial durchgehend zylindrisch konturiert ist.

Aus der CN 2009/60155 Y ist ein Kompressionsnagel mit zwei axial beabstandeten Gewindeabschnitten bekannt, wobei ein hinterer Gewindeabschnitt zylindrisch konturiert ist. Ein zwischen den Gewindeabschnitten vorgesehener Mittelabschnitt ist durchgehend zylindrisch konturiert. Auch hier ist die Kompressionswirkung verbesserungswürdig.

Aus der US 2,030,162 ist ein chirurgischer Kompressionsnagel bekannt, der ein vorderes Außengewinde und ein hinteres Außengewinde aufweist. Das hintere Außengewinde ist konisch konturiert und erstreckt sich bis unmittelbar zum vorderen Außengewinde (vgl. Fig. 22). Daneben sind aus der Druckschrift Kompressionsnägel bekannt, bei welchen das vordere und das hintere Gewinde über einen gewindefreien Zylinderabschnitt voneinander beabstandet sind.

Die US 5,964,768 A zeigt einen Kompressionsnagel mit einem hinteren und einem vorderen Außengewindeabschnitt, die unmittelbar axial ineinander übergehen.

Ausgehend von dem vorgenannten Stand der Technik liegt der Erfindung die Aufgabe zugrunde, einen chirurgischen Mittelfuß-Kompressionsnagel mit verbesserter Kompressionswirkung anzugeben. Der Kompressionsnagel soll gut und dauerhaft in den Fußknochen verankerbar sein und sich bevorzugt auf einfache Weise wieder ausdrehen lassen.

Diese Aufgabe wird mit den Merkmalen des Anspruchs 1 gelöst.

Vorteilhafte Weiterbildungen der Erfindung sind in den Unteransprüchen angegeben.

Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale als vorrichtungsgemäß offenbart gelten und beanspruchbar sein.

Zur Verbesserung der Kompression von mit dem Mittelfuß-Kompressionsnagel durchsetzten Fußknochen schlägt die Erfindung vor, das hintere Außengewinde mit einer konischen Hüllkontur zu versehen, wobei sich die konische Hüllkontur des hinteren Außengewindes in Richtung eines vorderen Endes des Kompressionsnagels verjüngt. Besonders bevorzugt ist es dabei, dass, zumindest ein maximaler, Außendurchmesser des hinteren Außengewindes größer ist als ein maximaler Außendurchmesser des vorderen Außengewindes, wobei noch weiter bevorzugt zusätzlich auch ein minimaler Außendurchmesser des hinteren Außengewindes größer ist als der maximale Außendurchmesser des vorderen Außengewindes. Aufgrund der konischen Hüllkontur des hinteren Außengewindes kann überraschend eine verbesserte Kompressionswirkung erzielt werden. Darüber hinaus kann ein selbsttätiges Verdrehen des Kompressionsnagels in eine Eindrehrichtung aufgrund der konischen Hüllkontur sicher vermieden werden, so dass die Implantationsposition (Fixationsposition) deutlich definierter ist als bei bekannten Fuß-Kompressionsnägeln.

Der erfindungsgemäße chirurgische Mittelfuß-Kompressionsnagel eignet sich zur Einbringung in den ersten (größten) Strang eines menschlichen Fußes, wobei der Kompressionsnagel ausgelegt ist um retrograd über Talus, Naviculare, Cuneiforme sowie Metatarsale in den Fuß implantiert bzw. eingedreht zu werden. Besonders zweckmäßig ist es dabei, wenn der Kompressionsnagel weiterbildungsgemäß im Kernbereich eine später noch zu erläuternde Durchgangsöffnung zur Aufnahme eines Kirschnerdrahtes aufweist, da dies ein revolutionäres geführtes Eindrehen des Kompressionsnagels mit Hilfe eines Zielgerätes über einen Kirschnerdraht ermöglicht.

Eine besonders gute Stabilität der Verbindung aus Kompressionsnagel und Fußknochen sowie eine optimierte Kompressionswirkung kann efindungsgemäß dadurch erzielt werden, dass der gewindefreie, zwischen den beiden Außengewindeabschnitten angeordnete, Mittelabschnitt ebenfalls, zumindest abschnittsweise, konisch konturiert ist und sich, wie die Hüllkonturen der Außengewinde in Richtung des vorderen Endes des Kompressionsnagels verjüngt. Durch die zumindest teilweise konusförmige Ausgestaltung des Mittelabschnittes, bevorzugt zumindest in einem vorderen Bereich, oder alternativ zumindest näherungsweise über seine gesamte Längserstreckung, ist es auch möglich eine Kompression von Fußknochenstücken zu bewirken, selbst wenn ein Zwischenknochenteilstück entfernt werden muss, da die Lage der verbleibenden Knochenstücke aufgrund der zumindest abschnittsweisen Konusform des Mittelabschnittes bzw. der korrespondierenden konusförmigen Bohrung vorgegeben bzw. definiert ist. Ganz besonders bevorzugt ist es, wenn der Mittelabschnitt nicht durchgehend konisch konturiert ist (was wie erwähnt alternativ möglich ist), sondern wenn zwischen einem konisch konturierten (vorderen) Mittelabschnitt und dem hinteren Außenabschnitt ein zylindrisch konturierter Bereich vorgesehen ist, der bevorzugt eine Axialerstreckung aus einem Wertebereich zwischen 10 mm und 30 mm, bevorzugt von etwa 20 mm aufweist. Besonders zweckmäßig ist es, wenn der Durchmesser dieses Zylinderabschnitts zumindest näherungsweise 6 mm beträgt. Ganz besonders bevorzugt bildet der vorgenannte Zylinderabschnitt den größten Durchmesserbereich bzw. hat den größten Durchmesser des Mittelabschnittes, welcher von dem Außengewinde überragt ist. Die zylindrische Ausbildung eines Teilabschnittes des Mittelabschnittes verleiht dem Kompressionsnagel mehr Stabilität, wodurch dieser verbessert gegen ein mögliches Durchbiegen gesichert ist bzw. entsprechenden Kräften und Momenten besser entgegenwirken kann.

An Stelle einer durchgehenden konischen Konturierung des Kernbereichs ist es von besonderem Vorteil, wenn der bevorzugt einteilige bzw. aus einem Stück gefertigte Kernbereich einen weiter vorne liegenden konischen Abschnitt und einen hinteren konischen Abschnitt aufweist, die über einen Zylinderabschnitt einteilig miteinander verbunden sind, wobei der Zylinderabschnitt bevorzugt Teil des Mittelabschnittes ist und noch weiter bevorzugt sich zwischen einem vorderen konischen Abschnitt des Mittelabschnittes und dem hinteren Außengewindeabschnitt befindet. Ganz besonders zweckmäßig ist es, wenn der vorderste Abschnitt des Kernbereichs, der an den konischen vorderen Abschnitt des Mittelabschnitts angrenzt, zylindrisch konturiert ist.

Bei der Operation wird der Fuß des Patienten bevorzugt seitlich gelagert. Eine Erreichbarkeit von Ferse und Innenfuß ist aufgrund der retrograden Einbringmöglichkeit nicht notwendig. Bevorzugt wird zur Implantation, geführt über den Kirschnerdraht mithilfe des Zielgerätes, eine Kernlochbohrung für den Kompressionsnagel im Fuß realisiert, insbesondere bis zu einem Abstand, bevorzugt von etwa 1cm, vor einen distalen Fixationsblock. Die benötigte Schraubenlänge kann dann bevorzugt an einer entsprechenden Skala oder auf sonstige Weise am Zielgerät und/oder einer entsprechenden Markierung am Bohrer abgelesen werden. Daraufhin erfolgt bevorzugt das Ausdrehen des Kernlochbohrers und es wird mittels eines Kopfraumfräsers, vorzugsweise per Hand, insbesondere am Talus ein Kopfraum zur Aufnahme des, vorzugsweise durchmessererweiterten, hinteren Außengewindes gefräst. Bevorzugt wird nach dem Entfernen des Kernbohrers, insbesondere mittels Reibahls, die zunächst zylindrische Bohrung konisch konturiert, um optimal mit der bevorzugten, zumindest abschnittsweisen, Konusform des erfindungsgemäßen Kompressionsnagels zusammenzuwirken, um hierdurch wiederum eine noch deutlich verbesserte Kompression zu erreichen. Daraufhin wird, bevorzugt über den Kirschnerdraht ein entsprechend abgemessener (passender) Kompressionsnagel eingeschraubt, woraufhin der Wundverschluss erfolgen kann.

Ganz besonders bevorzugt wird ein Sortiment unterschiedlich langer Kompressionsnägel, insbesondere in den Maßen 120 mm, 130 mm und 140 mm bereitgestellt. Als besonders vorteilhaft hat es sich herausgestellt, wenn die zylindrische Kernlochbohrung zur Aufnahme mindestens eines 2.0 Kirschnerdrahtes ausgelegt bzw. geeignet. ist.

Als besonders vorteilhaft hat es sich herausgestellt, wenn die Steigung des vorderen Gewindeabschnittes die Steigung des hinteren Gewindeabschnittes um einen Wertebereich zwischen 0,2 und 0,6, vorzugsweise zwischen 0,3 und 0,4 übersteigt. Ganz besonders zweckmäßig ist es, wenn die Steigung des vorderen Gewindes um 0,31 größer ist als die des hinteren Außengewindes. Besonders zweckmäßig ist es, wenn die Steigung des vorderen Außengewindes aus einem Wertebereich zwischen 1,8 und 2,2, vorzugsweise von 2,0 gewählt ist und/oder die Steigung des hinteren Außengewindes aus einem Wertebereich zwischen 1,4 und 1,8, vorzugsweise von 1,59. Im Hinblick auf eine weitere vorteilhafte Ausgestaltung der Erfindung hat es sich als vorteilhaft erwiesen, wenn der Winkel zwischen zwei einander zugewandten Gewindeflanken des vorderen und/oder hinteren Außengewindes aus einem Wertebereich zwischen 25° und 35°, vorzugsweise von etwa 30° gewählt ist. Weiter bevorzugt ist es, wenn die Axialerstreckung des vorderen Außengewindeabschnittes und/oder des hinteren Außengewindeabschnittes mindestens 10 mm beträgt. Ganz besonders bevorzugt ist die Axialerstreckung des jeweiligen Außengewindeabschnittes aus einem Wertebereich zwischen 10 mm und 30 mm gewählt und beträgt noch weiter bevorzugt etwa 20 mm. Grundsätzlich ist es möglich, nicht nur das hintere Außengewinde, sondern auch das vordere Außengewinde mit einer konischen Hüllkontur zu versehen, wobei es in diesem Fall dann bevorzugt ist, wenn die Hüllkonturen des vorderen und hinteren Außengewindes Teil einer gemeinsamen (gedachten) Hüllkontur mit einem einzigen (gemeinsamen) Konuswinkel sind. Als besonders vorteilhaft hat es sich jedoch herausgestellt, wenn das vordere Außengewinde keine konische Hüllkontur aufweist, sondern eine zylindrische Hüllkontur. Bevorzugt ist auch der Kernbereich radial innerhalb des vorderen Außengewindes zylindrisch und nicht konisch konturiert. Durch die zylindrische Hüllkonturierung des vordern Aussengwindes wird dem Kompressionsnagel mehr Stabilität verliehen, so dass der Gefahr eines möglichen Verbiegens des vorderen Abschnittes des Kompressionsnagels entgegengewirkt wird. Insgesamt wird somit eine dauerhaft stabile Positionierung ermöglicht.

Bevorzugt ist der Konuswinkel (Winkel zwischen zwei diametral gegenüberliegenden gedachten Hüllmantelfläche) der Hüllkontur des hinteres Außengewindes und/oder des vorderen Außengewindes aus einem Winkelbereich zwischen etwa 0,5° und etwa 4°, vorzugsweise zwischen etwa 1°und 3°, besonders bevorzugt von etwa 2° gewählt ist.

Bevorzugt entspricht der Außendurchmesser des Mittelabschnittes, in einem von dem vorderen Gewinde beabstandeten Bereich, dem maximalen Außendurchmesser des vorderen Außengewindes oder der Mittelabschnitt hat in einem von dem vorderen Außengewinde beabstandeten Bereich einen größeren Außendurchmesser als der maximale Außendurchmesser des vorderen Außengewindes. Vorgenannter, von dem vorderen Außengewinde beabstandeter Bereich kann von einem Konusabschnitt des Mittelabschnittes gebildet sein und/oder, falls vorhanden, von einem Zylinderabschnitt des Mittelabschnittes.

Bevorzugt überragen die beiden Au ßengewinde einen jeweils benachbarten Abschnitt des Mittelabschnitts (deutlich) in radialer Richtung, um hiermit im Vergleich zu bekannten Kompressionsnägeln eine verbesserte Verankerung im Fußknochen und damit schlussendlich eine verbesserte Kompression zu bewirken. Als besonders zweckmäßig hat es sich herausgestellt, wenn das hintere Außengewinde, zumindest in einem Abschnitt einen größten Durchmesser des bevorzugt, jedoch nicht zwingend konischen Mittelabschnittes, um einen Wert aus einem Wertebereich (gemessen in eine radiale Richtung) zwischen etwa 2,0 und etwa 7,0 mm, vorzugsweise zwischen etwa 3,0 und etwa 5,0 mm, besonders bevorzugt von etwa 3,3 mm überragt. Besonders zweckmäßig ist es, wenn das hintere Außengewinde den größten Durchmesser des Mittelabschnittes über den Großteil der Längenerstreckung des hinteren Außengewindes um eine Strecke aus einem vorgenannten Wertebereich überragt, wobei noch weiter bevorzugt dieser größte Durchmesser des Mittelabschnitts von dem vorerwähnten Zylinderabschnitt gebildet ist. Alternativ oder bevorzugt zusätzlich zu der zuvor erläuterten Ausgestaltung des hinteren Außengewindes ist es bevorzugt, wenn das vordere Außengewinde, bevorzugt über den größten Teil seiner Axialerstreckung, zumindest einen kleinsten Durchmesser des Mittelabschnittes um eine Strecke (gemessen in eine Radialrichtung) aus einem Wertebereich zwischen etwa 1,0 mm und etwa 4,0 mm vorzugsweise zwischen 1,5 mm und etwa 2,5 mm überragt.

Als besonders vorteilhaft hat es sich zur Erzielung eines optimalen und dauerhaften Haltes im Knochen sowie einer damit einhergehenden, dauerhaft guten Kompression herausgestellt, wenn die Gewindetiefe von Gewinderillen des vorderen Außengewindes und/oder des hinteren Außengewindes über mindestens 75%, vorzugsweise mindestens 85%, bevorzugt über mindestens 95% der Axialerstreckung des entsprechenden Außengewindes konstant ist. Mit anderen Worten ist es also bevorzugt, wenn der radial innerhalb der Gewinderillen angeordnete Kernbereich bei dem hinteren Außengewinde (wie die Hüllkontur dieses Außengewindes) konisch konturiert ist, insbesondere mit dem Konuswinkel des hinteren Außengewindes.

Bevorzugt entspricht der Konuswinkel des konischen Teils des Mittelabschnitts dem Konuswinkel der Hüllkontur des hinteren Außengewindes.

Um ein erleichtertes Eindrehen des Kompressionsnagels zu ermöglichen bzw. um auf einen separaten Gewindeschneidschritt vor dem Eindrehen des Kompressionsnagels verzichten zu können, ist in Weiterbildung der Erfindung mit Vorteil vorgesehen, dass dem vorderen Außengewinde vordere Furch- und/oder Schneidmittel und/oder dem hinteren Außengewinde hintere Furch- und/oder Schneidmittel zum selbsttätigen Gewindeformen zugeordnet sind. Die Furch- und/oder Schneidmittel können auch, insbesondere gleichzeitig, dazu dienen, ein erleichtertes Lösen des Kompressionsnagels zu ermöglichen, indem die Furchen- und/oder Schneidmittel mit der Zeit nachgewachsenen Knochen, Knorpel oder sonstigem Gewebe selbsttätig freischneiden können. Ein größerer, komplexer und komplikationsbehafteter operativer Eingriff zum Entfernen des Kompressionsnagels ist aufgrund der weiterbildungsgemäßen Ausgestaltungsform verzichtbar. Es reicht aus, den Fuß nur in einem hinteren Bereich zu öffnen. Furch-und/oder Schneidmittel sind bevorzugt integral im entsprechenden Außengewinde vorgesehen. Insbesondere indem entsprechende, in Umfangsrichtung wirkende Schneidkanten vorgesehen sind. Bevorzugt sind mehrere, insbesondere gleichmäßig in Umfangsrichtung beabstandete Schneidkanten an zumindest einem der Außengewinde realisiert, insbesondere an einem in Eindrehrichtung gelegenen Ende. Auch diese können dann ein erleichtertes Lösen des Kompressionsnagels ermöglichen.

Wie eingangs bereits angedeutet ist es besonders zweckmäßig, wenn im, vorzugsweise aus Titan ausgebildeten, massiven Kernbereich des, insbesondere einteiligen Kompressionsnagels eine zentrische Durchgangsöffnung zum Durchführen eines Kirschnerdrahtes zum Führen des Kompressionsnagel beim Eindrehen und/oder Ausdrehen vorgesehen ist. Dabei ist der Durchmesser bevorzugt aus einem Wertebereich zwischen 2,05 und 2,5 mm gewählt, beträgt jedenfalls bevorzugt mehr als 2,0 mm. Ganz besonderes bevorzugt beträgt der Innendurchmesser etwa 2,2 mm.

Die Erfindung führt auch auf ein System umfassend ein nach dem Konzept der Erfindung ausgebildeten, zuvor beschriebenen Kompressionsnagel sowie ein Zielgerät als Hilfestellung bei der Implantation des Kompressionsnagels. Bevorzugt ist das Zielgerät ausgelegt zur Aufnahme unterschiedlicher Führungen, beispielsweise für einen Kirschnerdraht oder für einen Kernlochbohrer, wobei diese Führungen noch weiter bevorzugt mittels eines Bajonettverschlusses festlegbar sind.

Weitere Vorteile, Merkmale und Einzelheiten der Erfindung ergeben sich aus der folgenden Beschreibung bevorzugter Ausführungsbeispiele sowie anhand der Zeichnungen.

Diese zeigen in:
- Fig. 1: eine Seitenansicht eines bevorzugten Ausführungsbeispiels eines erfindungsgemäßen chirurgischen Mittelfuß-Kompressionsnagels,
- Fig. 2: eine Längsschnittansicht des Kompressionsnagels gemäß Fig. 1 entlang der Schnittlinie A-A,
- Fig. 3: eine Rückansicht des Kompressionsnagels gemäß Fig. 1,
- Fig. 4: eine Vorderansicht des Kompressionsnagels gemäß Fig. 1,
- Fig. 5: eine Detailvergrößerung des Details L gemäß Fig. 3,
- Fig. 6: eine Detailvergrößerung des Details B aus Fig. 2,
- Fig. 7: eine Detailvergrößerung des Details C aus Fig. 2,
- Fig. 8: eine Querschnittsansicht entlang der Schnittlinie K-K gemäß Fig. 1, und
- Fig. 9: eine Querschnittsansicht entlang der Schnittlinie E-E gemäß Fig. 1.

In den Figuren ist ein chirurgischer Mittelfuß-Kompressionsnagel 1, im Folgenden als Kompressionsnagel 1 dargestellt. Dieser weist eine Länge l in dem gezeigten Ausführungsbeispiel von 120 mm auf, wobei bevorzugt in einem Sortiment unterschiedlich lange Kompressionsnägel zusammengefasst sind. Der Kompressionsnagel 1 umfasst einen massiven Kernbereich 2, der sich axial von einem in der Zeichnungsebene links dargestellten vorderen Ende 3 bis zu einem in der Zeichnungsebene rechten hinteren Ende 4 des Kompressionsnagels 1 erstreckt. Der massive Kernbereich 1 ist mit einer Durchgangsöffnung 5 zur Aufnahme eines 2.0 Kirschnerdrahtes versehen, wobei die zylindrische Durchgangsöffnung 5 einen Durchmesser d1 von in dem gezeigten Ausführungsbeispiel 2,2 mm aufweist.

An das vordere Ende 3 grenzt axial ein vorderer Gewindeabschnitt 6 an, mit einer Axialerstreckung in dem gezeigten Ausführungsbeispiel von 20 mm. Der vordere Gewindeabschnitt 6 weist ein vorderes Außengewinde 7 auf, welches radial an den in dem vorderen Bereich zylindrischen Kernbereich 2 anschließt. Das vordere Außengewinde 7 weist in dem gezeigten Ausführungsbeispiel eine Steigung von 2,0 mm auf und ist mit einer zylindrischen Hüllkontur versehen. Dabei weist das vordere Außengewinde 7 axial durchgängig einen Außendurchmesser d2 von 7 mm auf. Das vordere Außengewinde 7 überragt einen geringsten Außendurchmesser eines axial an den vorderen Gewindeabschnitt 6 angrenzenden Mittelabschnitt 8. Dieser geringste Außendurchmesser d3 beträgt in dem gezeigten Ausführungsbeispiel etwa 4,5 mm.

Der Mittelabschnitt 8 ist gewindefrei ausgebildet und erstreckt sich axial zwischen dem vorderen (zylindrischen) Gewindeabschnitt 6 und einem hinteren Gewindeabschnitt 9, der ebenfalls eine Axialerstreckung von 20 mm aufweist und der mit einem hinteren Außengewinde 10 versehen ist. Der Mittelabschnitt 8 teilt sich auf sich in einem vorderen, konisch konturierten Konusabschnitt 11 und einem axial daran angrenzenden hinteren Zylinderabschnitt 12, wobei der Zylinderabschnitt eine Axialerstreckung 11 von in dem gezeigten Ausführungsbeispiel 20 mm aufweist und einem Außendurchmesser d4 von 6mm. Der Außendurchmesser d4 stellt gleichzeitig den größten Durchmesser des Mittelabschnittes 8 dar. Zu erkennen ist, dass der Mittelabschnitt 8 über mehr als dreiviertel seiner Axialerstreckung konisch konturiert ist.

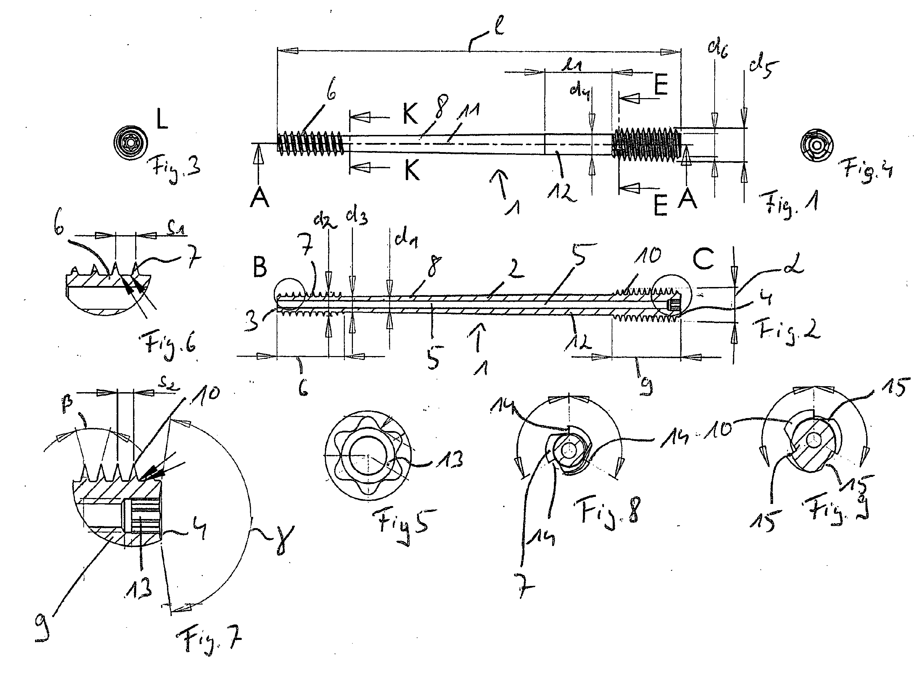

Das hintere Außengewinde 10 weist eine konische Hüllkontur auf, mit einem Konuswinkel α von in dem gezeigten Ausführungsbeispiel 2°. Die Gewindetiefe des hinteren Außengewindes 10 ist in dem gezeigten Ausführungsbeispiel über nahezu seiner gesamten Axialerstreckung konstant, da auch der Kernbereich im Bereich des hinteren Gewindeabschnittes 9 konisch konturiert ist und einem dem Konuswinkel α entsprechenden Konuswinkel von 2° aufweist. Wie sich aus Fig. 1 ergibt, beträgt der maximale Außendurchmesser d5 des Kompressionsnagels 1 und gleichzeitig des hinteren Außengewindes 10 im Bereich des hinteren Endes 4 10 mm. In diesem Bereich überragt somit das Außengewinde den größten Durchmesser d4 des Mittelabschnittes um 4 mm und den Kernbereich um 3,29 mm, welcher in diesem Bereich einen Durchmesser d6 von 6,71 mm aufweist.

Aus Fig. 7 ist zu erkennen, dass die Steigung s2 des hinteren Außengewindes 10 in dem gezeigten Ausführungsbeispiel 1,59 beträgt. Ein Winkel β zwischen zwei einander zugewandten Gewindeflanken des hinteren Außengewindes beträgt 30°. Zu erkennen ist auch, dass das hintere Ende 4 des Kompressionsnagels 1 stirnseitig konkav konturiert ist und einen Krümmungswinkel γ von 163° aufweist. Abweichende Krümmungswinkel sind alternativ realisierbar. In Fig. 5 ist ein in Fig. 7 im Schnitt dargestellter Antrieb 13, welcher sich im Bereich des hinteren Endes 4 befindet in einer Draufsicht gezeigt. Zu erkennen ist, dass der Antrieb 13 beispielhaft als Torx-Antrieb ausgebildet ist.

In Fig. 8 sind drei gleichmäßig in Umfangsrichtung beabstandete Schneidkanten 14 des als selbstschneidendes Gewinde ausgebildeten vorderen Gewindes 6 zu erkennen. Die Schneidkanten 14 bilden Schneidmittel, um beim Eindrehen in den Knochen selbsttätig ein Gegengewinde zu formen. In analoger Weise sind dem hinteren Außengewinde 10 hintere Schneidmittel in Form von drei gleichmäßig in Umfangsrichtung beabstandeten Schneidkanten 15 zugeordnet, wobei bei beiden Schneidmitteln Schneidkanten für jede Drehrichtung vorgesehen sind, um auch ein erleichtertes Lösen des Kompressionsnagels 1 zu ermöglichen.

### Bezugszeichenliste

- 1: Kompressionsnagel
- 2: Kernbereich
- 3: vorderes Ende
- 4: hinteres Ende
- 5: Durchgangsöffnung
- 6: vorderer Gewindeabschnitt
- 7: vorderes Außengewinde
- 8: Mittelabschnitt
- 9: hinterer Gewindeabschnitt
- 10: hinteres Außengewinde
- 11: Konusabschnitt
- 12: Zylinderabschnitt
- 13: Antrieb
- 14: Schneidkanten
- 15: Schneidkanten

- d1: Durchmesser
- d2: Durchmesser
- d3: Durchmesser
- d4: Durchmesser
- d5: Durchmesser
- d6: Durchmesser

- l: Länge
- l1: Längenerstreckung

- α: Konuswinkel des hinteren Außengewindes
- β: Flankenwinkel
- γ: Konkavwinkel
- s1: Steigerung des vorderen Gewindeabschnittes
- s2: Steigerung des hinteren Gewindeabschnittes

## Patentansprüche

1. Chirurgischer Mittelfuß-Kompressionsnagel (1) mit einem einen Antrieb aufweisenden Kernbereich (2), einem vorderen Gewindeabschnitt (6) mit einem vorderen Außengewinde (7), einem hinteren Gewindeabschnitt (9) mit einem hinteren Außengewinde (10) und mit einem zwischen den Gewindeabschnitten angeordneten Mittelabschnitt (8), wobei die Gewindesteigung des vorderen Außengewindes (s1) zum Erzielen einer Kompression größer ist als diejenige (s2) des hinteren Außengewindes (10), wobei das hintere Außengewinde (10) eine konische Hüllkontur aufweist, die sich in Richtung des vorderen Endes (3) des Kompressionsnagels (1) verjüngt, wobei der Mittelabschnitt (8) zumindest abschnittsweise konisch konturiert ist und sich in Richtung des vorderen Endes (3) des Kompressionsnagels (1) verjüngt,
**dadurch gekennzeichnet,**
**dass** der Mittelabschnitt (8) gewindefrei ist.

2. Kompressionsnagel nach Anspruch 1,
**dadurch gekennzeichnet,**
**dass** axial benachbart zu einem konischen Axialabschnitt des Mittelabschnitts, ein zylindrischer Axialabschnitt des Mittelabschnitts vorgesehen ist.

3. Kompressionsnagel nach einem der Ansprüche 1 oder 2,
**dadurch gekennzeichnet,**
**dass** das vordere Außengwinde (7) eine zylindrische oder konische Hüllkontur aufweist.

4. Kompressionsnagel (1) nach einem der Ansprüche 1 oder 2,
**dadurch gekennzeichnet,**
**dass** die konische Hüllkontur des hinteren Außengewindes (10) einen Konuswinkel aus einem Winkelbereich zwischen 0,5° und 4°, vorzugsweise zwischen 1° und 3°, bevorzugt von 2° aufweist.

5. Kompressionsnagel nach einem der vorherigen Ansprüche,
**dadurch gekennzeichnet,**
**dass** der Mittelabschnitt (8) über den größten Teil seiner Axialerstreckung, bevorzugt über seine gesamte Axialerstreckung, konisch konturiert ist.

6. Kompressionsnagel (1) nach Anspruch 4,
**dadurch gekennzeichnet,**
**dass** sich das vordere Außengewinde (7) und das hintere Außengewinde (10) in radialer Richtung über den Mittelabschnitt (8) hinaus erstrecken.

7. Kompressionsnagel (1) nach Anspruch 6,
**dadurch gekennzeichnet,**
**dass** sich das hintere Außengewinde (10) in radialer Richtung um eine Strecke aus einem Wertebereich zwischen 2,0 mm und 7,0 mm, vorzugsweise zwischen 3,0 und 5,0 mm, bevorzugt von 3,3 mm über einen größten Durchmesser des Mittelabschnitts (8) erstreckt und/oder dass sich das vordere Außengewinde (7) in radialer Richtung um eine Strecke aus einem Wertebereich zwischen 1,0 mm und 4,0 mm, vorzugsweise zwischen 1,5 mm und 2,5 mm über einen kleinsten Durchmesser des Mittelabschnitts (8) erstreckt.

8. Kompressionsnagel (1) nach einem der vorhergehenden Ansprüche,
**dadurch gekennzeichnet,**
**dass** eine Gewindetiefe von Gewinderillen des vorderen Außengewindes (7) und/oder des hinteres Außengewindes (10) über mindestens 75%, vorzugsweise mindestens 85%, bevorzugt über mindestens 95% der Axialerstreckung des vorderen bzw. hinteren Außengewindes (7, 10) konstant ist.

9. Kompressionsnagel (1) nach einem der vorhergehenden Ansprüche,
**dadurch gekennzeichnet,**
**dass** sich der vom vorderen bis zum hinteren Ende (3, 4) des Kompressionsnagels (1) erstreckende Kernbereich (2), zumindest abschnittsweise, konisch konturiert ist, und bevorzugt mindestens einen, insbesondere gewindefreien, Zylinderabschnitt (12) aufweist, wobei der Zylinderabschnitt bevorzugt Teil des Mittelabschnittes (8) ist und noch weiter bevorzugt ein hinteres Ende des Mittelabschnittes (8) bildet.

10. Kompressionsnagel (1) nach einem der vorhergehenden Ansprüche,
**dadurch gekennzeichnet,**
**dass** dem vorderen Außengewinde (7) vordere Furch- und/oder Schneidmittel zum selbsttätigen Gewindeformen im Fußknochen beim Eindrehen in eine und/oder Ausdrehen aus einer Fußbohrung zugeordnet sind und/oder dass dem hinteren Außengewinde (10) hintere Furch- und/oder Schneidmittel zum selbsttätigen Gewindeformen im Fußknochen beim Eindrehen in eine und/oder Ausdrehen aus einer Fußbohrung zugeordnet sind.

11. Kompressionsnagel (1) nach einem der vorhergehenden Ansprüche,
**dadurch gekennzeichnet, dass**
im Kernbereich (2) eine zentrische Durchgangsöffnung (5) zum Durchführen eines Kirschnerdrahtes zum Führen des Kompressionsnagels (1) beim Eindrehen und/oder Ausdrehen vorgesehen ist.

12. System, umfassend einen Kompressionsnagel (1) nach einem der vorhergehenden Ansprüche sowie ein Zielgerät für die Implantation des Kompressionsnagels (1).

## Claims

1. A surgical metatarsal compression nail (1) with a core region (2) having a drive, with an anterior threaded section (6) having an anterior outside thread (7), with a posterior threaded section (9) having a posterior outside thread (10) and with a central section (8) situated between the threaded sections, wherein the pitch of the thread of the anterior outside thread (s1) is greater than that (s2) of the posterior outside thread (10) in order to achieve a compression, wherein the posterior outside thread (10) has a conical enveloping contour, which tapers in the direction of the anterior end (3) of the compression nail (1), wherein the central section (8) is contoured conically in at least some sections and tapers in the direction of the anterior end (3) of the compression nail (1),
**characterized in that**
the central section (8) is without a thread.

2. The compression nail according to Claim 1,
**characterized in that**
a cylindrical axial section of the central section is provided in axial proximity to a conical axial section of the central section.

3. The compression nail according to any one of Claims 1 or 2,
**characterized in that**
the anterior outside thread (7) has a cylindrical or conical enveloping contour.

4. The compression nail (1) according to any one of Claims 1 of 2,
**characterized in that**
the conical enveloping contour of the posterior outside thread (10) has a cone angle of an angle range between 0.5° and 4°, preferably between 1 ° and 3°, more preferably 2°.

5. The compression nail according to any one of the preceding claims,
**characterized in that**
the central section (8) is conically contoured over most of its axial extent, preferably over its entire axial extent.

6. The compression nail (1) according to Claim 4,
**characterized in that**
the anterior outside thread (7) and the posterior outside thread (10) extend beyond the central section (8) in the radial direction.

7. The compression nail (1) according to Claim 6,
**characterized in that**
the posterior outside thread (10) extends in the radial direction over the largest diameter of the central section (8) for a segment from a value range between 2.0 mm and 7.0 mm, preferably between 3.0 and 5.0 mm, more preferably 3.3 mm, and/or the anterior outside thread (7) extends in the radial direction by a distance from an angle range between 1.0 mm and 4.0 mm, preferably between 1.5 mm and 2.5 mm over the smallest diameter of the central section (8) in the radial direction.

8. The compression nail (1) according to any one of the preceding claims,
**characterized in that**
the depth of the thread in the threaded grooves of the anterior outside thread (7) and/or the posterior outside thread (10) is constant over at least 75%, preferably at least 85%, more preferably over at least 95% of the axial extent of the anterior and/or posterior outside thread (7, 10).

9. The compression nail (1) according to any one of the preceding claims,
**characterized in that**
the core region (2) extending from the anterior to the posterior ends (3, 4) of the compression nail (1) is contoured conically in at least some sections and preferably has at least one cylindrical section (12) that is free of thread, wherein the cylindrical section is preferably part of the central section (8) and even more preferably forms a posterior end of the central section (8).

10. The compression nail (1) according to any one of the preceding claims,
**characterized in that**
the anterior grooving and/or cutting means for automatic thread-cutting in the foot bone when screwed into and/or out of a foot bore are assigned to the anterior outside thread (7) and/or posterior grooving and/or cutting means for automatic thread-cutting in the foot bone when screwing in and/or unscrewing from a foot borehole are allocated to the posterior outside thread (10).

11. The compression nail (1) according to any one of the preceding claims,
**characterized in that**
a central through-opening (5) for passing a Kirschner wire through the opening for guiding the compression nail (1) when screwed in and/or unscrewed is provided in the core region (2).

12. A system comprising a compression nail (1) according to any one of the preceding claims as well as a targeting device for implantation of the compression nail (1).

## Revendications

1. Clou chirurgical de compression du métatarse (1) comprenant une région de coeur (2) présentant un entraînement, une section filetée avant (6) avec un filetage extérieur avant (7), une section filetée arrière (9) avec un filetage extérieur arrière (10) et une section centrale (8) disposée entre les sections filetées, le pas de filetage du filetage extérieur avant (s1), pour réaliser une compression, étant plus grand que celui (s2) du filetage extérieur arrière (10), le filetage extérieur arrière (10) présentant un contour d'enveloppe conique qui se rétrécit dans la direction de l'extrémité avant (3) du clou de compression (1), la section centrale (8) ayant au moins en partie un contour conique et se rétrécissant dans la direction de l'extrémité avant (3) du clou de compression (1),
**caractérisé en ce que**
la section centrale (8) est exempte de filetage.

2. Clou de compression selon la revendication 1,
**caractérisé en ce**
**qu'**une section axiale cylindrique de la section centrale est prévue axialement à côté d'une section axiale conique de la section centrale.

3. Clou de compression selon l'une quelconque des revendications 1 ou 2,
**caractérisé en ce que**
le filetage extérieur avant (7) présente un contour d'enveloppe cylindrique ou conique.

4. Clou de compression (1) selon l'une quelconque des revendications 1 ou 2,
**caractérisé en ce que**
le contour d'enveloppe conique du filetage extérieur arrière (10) présente un angle de conicité dans une plage angulaire comprise entre 0,5° et 4°, de préférence entre 1 ° et 3°, de préférence de 2°.

5. Clou de compression selon l'une quelconque des revendications précédentes,
**caractérisé en ce que**
la section centrale (8) a un contour conique sur la majeure partie de son étendue axiale, de préférence sur toute son étendue axiale.

6. Clou de compression (1) selon la revendication 4,
**caractérisé en ce que**
le filetage extérieur avant (7) et le filetage extérieur arrière (10) s'étendent dans la direction radiale au-delà de la section centrale (8).

7. Clou de compression (1) selon la revendication 6,
**caractérisé en ce que**
le filetage extérieur arrière (10) s'étend dans la direction radiale sur une distance dans une plage de valeurs comprise entre 2,0 mm et 7,0 mm, de préférence entre 3,0 et 5,0 mm, de préférence de 3,3 mm, sur un plus grand diamètre de la section centrale (8) et/ou **en ce que** le filetage extérieur avant (7) s'étend dans la direction radiale sur une distance dans une plage de valeurs comprise entre 1,0 mm et 4,0 mm, de préférence entre 1,5 mm et 2,5 mm, sur un plus petit diamètre de la section centrale (8).

8. Clou de compression (1) selon l'une quelconque des revendications précédentes,
**caractérisé en ce**
**qu'**une profondeur de filetage des rainures de filetage du filetage extérieur avant (7) et/ou du filetage extérieur arrière (10) est constante sur au moins 75 %, de préférence sur au moins 85 %, de préférence sur au moins 95 %, de l'étendue axiale du filetage extérieur avant, respectivement arrière (7, 10).

9. Clou de compression (1) selon l'une quelconque des revendications précédentes,
**caractérisé en ce que**
la région de coeur (2) s'étendant depuis l'extrémité avant jusqu'à l'extrémité arrière (3, 4) du clou de compression (1) a un contour au moins en partie conique, et présente de préférence au moins une section cylindrique, notamment sans filetage (12), la section cylindrique faisant de préférence partie de la section centrale (8) et formant, de manière encore plus préférée, une extrémité arrière de la section centrale (8).

10. Clou de compression (1) selon l'une quelconque des revendications précédentes,
**caractérisé en ce que**
des moyens autotaraudeurs et/ou de découpe avant, pour la formation automatique de filetages dans l'os du pied lors du vissage dans un et/ou du dévissage hors d'un perçage dans le pied sont associés au filetage extérieur avant (7) et/ou **en ce que** des moyens autotaraudeurs et/ou de découpe arrière pour la formation automatique de filetages dans l'os du pied lors du vissage dans un et/ou du dévissage hors d'un perçage dans le pied sont associés au filetage extérieur arrière (10).

11. Clou de compression (1) selon l'une quelconque des revendications précédentes,
**caractérisé en ce que**
dans la région de coeur (2) est prévue une ouverture de passage centrale (5) pour le passage d'une broche de Kirschner pour guider le clou de compression (1) lors du vissage et/ou du dévissage.

12. Système comprenant un clou de compression (1) selon l'une quelconque des revendications précédentes, ainsi qu'un instrument de visée pour l'implantation du clou de compression (1).
